# EUROPEAN PATENT SPECIFICATION

(11) **EP 1 635 779 B1**
(45) Date of publication and mention of the grant of the patent: **09.12.2009**
(21) Application number: 04727287.7
(22) Date of filing: 14.04.2004
(51) Int. Cl.: A61K 8/42, A61Q 19/00, A61Q 19/08, A61Q 19/10

(54) **COSMETIC PEELING METHOD USING UREA**
KOSMETISCHES PEELING-VERFAHREN UNTER VERWENDUNG VON HARNSTOFF
TECHNIQUE DE PEELING COSMETIQUE AU MOYEN D'UREE

(30) Priority: 24.04.2003 FR 0305049; 18.06.2003 US 479162 P
(43) Date of publication of application: 22.03.2006
(73) Proprietor: L'ORÉAL, 75008 Paris (FR)
(72) Inventor: SORE, Gabrielle, F-75012 Paris (FR); HANSENNE, Isabelle, Westfield, NJ 07090 (US)
(74) Representative: Brohmi, Karim
(86) International application number: PCT/EP2004/004005
(87) International publication number: WO 2004/093838

(56) References cited:
- EP-A- 1 293 204
- WO-A-01/00167
- FR-A- 2 498 929
- US-A- 5 919 470

## Description

The present invention relates to a peeling process that uses a composition containing at least 20% by weight of urea in a physiologically acceptable medium.

Peelings are a well-known means for improving the surface appearance of the skin, in particular to attenuate pigmentation defects such as actinic lentigo or acne or chickenpox scars, or to smooth out the irregularities of the texture of the skin, in particular wrinkles and fine lines.

They have the effect of removing a part of the skin to be treated (epidermis and possibly the upper layer of the dermis) via chemical methods (especially glycolic acid or salicylic acid) .

Although the compositions used hitherto to perform chemical peelings have given satisfactory results, it nevertheless remains that they are not without side effects. Thus, peelings with salicylic acid may give rise to cases of salicylism in the event of an overdose or of prolonged application.

There is thus still a need for peeling compositions that are effective while at the same time being well tolerated.

The Applicant has now found that compositions comprising at least 20% by weight of urea are useful for this purpose.

Urea is a humectant and moisturizer that is conventionally used in small amount (less than 5% by weight) in cosmetic compositions. At higher concentration (greater than 10% by weight), urea has keratolytic properties resulting from its capacity to degrade corneodesmosine, which constitutes the cellular junctions between the epidermal keratinocytes, and also bacteriostatic properties.

Keratolytic compositions containing from 21% to 40% by weight of urea have thus been described as useful in application to the skin, in the treatment of dry skin (US-5 919 470). However, these compositions are applied in the form of a semi-solid cream that is intended to remain on the skin, and as such they give rise to stinging and an odour - resulting from the decomposition of the urea - that the user finds hard to tolerate. The user is thus liable to lengthen the application interval of these creams, or even to stop applying them, which is harmful to their long-term efficacy. In addition, the creams disclosed in the said document are difficult to remove by rinsing with water, and as such they would not be suitable for use as peeling compositions.

This is likewise the case for the gels, preferably containing from 10% to 20% by weight of urea, disclosed in document EP-1 293 204.

US-6 429 231 also discloses compositions containing from 0.1% to 40% by weight of urea, which may be applied in various galenical forms, in particular in the form of creams, gels, sticks or cleansing compositions, for treating various conditions such as acne, psoriasis, seborrhoea, rosacea and hyperpigmentations. As mentioned previously, the prolonged use of urea at high concentration in cream form is not desirable. The compositions illustrated in the said patent thus comprise an amount of urea in the region of 10% by weight. In addition, the time of contact with the skin of the cleansing compositions containing high concentrations of urea is too short to allow them to act. Thus, the said document does not disclose a sufficiently effective and well-tolerated method for attenuating the abovementioned skin conditions.

There was therefore nothing in the prior art to suggest that urea could be used at high concentration to perform chemical peelings for cosmetic purposes.

One subject of the invention is thus a cosmetic process for treating visible and/or tactile irregularities on human skin, comprising the steps consisting in:
(a) applying topically to the skin a composition comprising, in a physiologically acceptable medium, at least 20% by weight of urea,
(b) leaving the composition in contact with the skin for a time of between 5 minutes and 30 minutes, and
(c) removing the composition by rinsing.

The composition used in the present invention contains an amount of urea that depends on the depth of peeling that is to be performed, i.e. the layers of the epidermis or dermis that are to be removed, and for example an amount ranging from 20% to 50% by weight of urea, for example an amount of 20%, 30%, 40% or 50% by weight of urea relative to the total weight of the composition.

The composition according to the invention may be in any galenical form conventionally used for topical application, provided that it can be removed easily by rinsing, and especially in the form of an aqueous gel or an aqueous or aqueous-alcoholic solution. It may also, by adding a fatty or oily phase, be in the form of a dispersion of the lotion or emulsion type of liquid or semi-liquid consistency, preferably obtained by dispersing a fatty phase in an aqueous phase (O/W). These compositions are prepared according to the usual methods.

It may be applied by any means that allow uniform distribution, and especially using a pad of cottonwool, a rod, a brush, a gauze, a spatula or a swab, or alternatively by spraying, and may be removed by rinsing with water or with a mild detergent.

According to one preferred embodiment of the invention, the composition contains a continuous aqueous phase.

When the composition is in emulsion form, the proportion of the oily phase of the emulsion may range, for example, from 1% to 30% by weight and preferably from 5% to 20% by weight relative to the total weight of the composition. The oils, emulsifiers and co-emulsifiers used in the composition in emulsion form are chosen from those conventionally used in cosmetics or dermatology. The emulsifier and the co-emulsifier are generally present in the composition in a proportion ranging from 0.3% to 30% by weight and preferably from 0.5% to 20% by weight relative to the total weight of the composition. The emulsion may also contain lipid vesicles.

As fatty substances that may be used in the invention, it is possible to use oils and especially mineral oils (liquid petroleum jelly), oils of plant origin (avocado oil or soybean oil), synthetic oils (perhydrosqualene), silicone oils (cyclomethicone) and fluoro oils (perfluoropolyethers).

As emulsifiers and co-emulsifiers that may be used in the invention, examples that may be mentioned include fatty acid esters of polyethylene glycol, such as PEG-100 stearate, PEG-50 stearate and PEG-40 stearate; fatty acid esters of polyols, such as glyceryl stearate, sorbitan tristearate and oxyethylenated sorbitan stearates, which are available under the trade names Tween^{®} 20 or Tween^{®} 60, for example; and mixtures thereof.

The composition according to the invention may also contain adjuvants that are common in cosmetics and dermatology, such as thickeners, active agents, preserving agents, solvents and fillers. The amounts of these various adjuvants are those conventionally used in the fields under consideration, for example from 0.01% to 20% of the total weight of the composition. Depending on their nature, these adjuvants may be introduced into the fatty phase or into the aqueous phase. These adjuvants and concentrations thereof should be such that they do not harm the keratolytic properties of urea.

Thickeners that may be mentioned in particular include: xanthan gum, an optionally crosslinked acrylic acid homopolymer or copolymer, a polyacrylamide, an acrylamidomethylpropanesulphonic acid homopolymer or copolymer, and cellulose derivatives, including hydroxypropyl cellulose.

According to one preferred embodiment of the invention, the composition also contains at least one compound chosen from alcohols and polyols containing from 1 to 3 carbon atoms. Examples of such compounds that may be mentioned include ethanol, isopropanol, glycerol and propylene glycol.

As mentioned previously, this composition is intended to be used in a peeling process for attenuating the visible and/or tactile irregularities on the skin, and in particular for attenuating wrinkles and fine lines and/or pigmentation marks and/or scars - in particular acne scars - and/or for unblocking skin pores. The composition may thus be applied to the face and/or the neck and/or the neckline and/or the hands and/or the back.

To optimize its effects, the peeling process according to the invention preferably comprises additional steps of preparing the skin for peeling and/or of skincare after peeling using compositions containing smaller amounts of urea than the peeling composition described above.

Thus, it is preferable for the process according to the invention to comprise, besides the steps mentioned above:
- a preliminary step of applying to the skin a composition containing, in a physiologically acceptable medium, from 0.5% to 10% by weight of urea, before performing step (a), and/or
- an additional step of applying to the skin a composition containing, in a physiologically acceptable medium, from 0.5 to 10% by weight of urea, after performing step (c).

Performing the preliminary step above also makes it possible to detect any allergy to urea and to improve the efficacy and uniformity of the peeling.

The compositions used in these preliminary and additional steps may be applied in the morning and evening, for example, optionally in combination with a composition for protecting the skin against the effects of UV rays. The pretreatment composition may be applied for one to four weeks and the post-treatment composition may be applied for one day to eight weeks, for example.

The process according to the invention, including the optional preliminary and additional steps, may be performed only once or repeated up to five times, if necessary, the peeling sessions preferably being at intervals of from one to eight weeks.

The invention will now be illustrated with the non-limiting examples that follow. In these examples, the amounts are indicated as weight percentages.

### EXAMPLES

### Example 1: Peeling composition

| | | |
|---|---|---|
| Urea | | 25 % |
| Xanthan gum | | 1 % |
| Preserving agent | | qs |
| Ethanol | | 10 % |
| Purified water | qs | 100 % |

This composition is prepared in a manner that is conventional for those skilled in the art. It is intended to be applied to the face to smooth out wrinkles and fine lines, according to the following process.

The skin is first cleansed and degreased, for example using a gauze impregnated with ethanol or acetone, so as to remove the sebum and the dead cells and to harmonize the skin pH. The sensitive areas of the skin (corners of the lips, the nostrils and the corners of the eyes) are then protected with an occlusive ointment and the eyes themselves are protected with eye protection. The above composition is then applied to the skin, using a brush, as a thin coat. After leaving the composition on for a few minutes, it is removed by rinsing using a compress soaked with warm water.

### Example 2: Peeling composition

| | | |
|---|---|---|
| Urea | | 40 % |
| Glycerol | | 10 % |
| Propylene glycol | | 10 % |
| Preserving agent | | qs |
| Purified water | qs | 100 % |

This composition is prepared in a manner that is conventional for those skilled in the art. It is intended to be applied to the hands to attenuate age marks, according to the process described in Example 1.

### Example 3: Peeling composition

| | | |
|---|---|---|
| Urea | | 50 % |
| Preserving agent | | qs |
| Purified water | qs | 100 % |

This composition is prepared in a manner that is conventional for those skilled in the art. It is intended to be applied to the back to treat comedones, according to the process described in Example 1.

### Example 4: Peeling composition

| | | |
|---|---|---|
| Urea | | 30 % |
| 3-(N-Morpholino)propanesulphonic acid | | 10 % |
| Preserving agent | | qs |
| Sodium hydroxide | qs | pH 7 |
| Purified water | qs | 100 % |

This composition is prepared in a manner that is conventional for those skilled in the art. It is intended to be applied to the neckline to attenuate pigmentation marks, according to the process described in Example 1.

### Example 5: Peeling-preparation composition

| | | |
|---|---|---|
| Urea | | 10 % |
| Preserving agent | | qs |
| Carbomer | | 2 % |
| Sodium hydroxide | qs | pH 7 |
| Purified water | qs | 100 % |

This composition is prepared in a'manner that is conventional for those skilled in the art. It is intended to be applied morning and evening to precleansed skin, for one to four weeks, to prepare the skin for application of the peeling compositions illustrated in Examples 1 to 4.

## Claims

1. Cosmetic process for treating visible and/or tactile irregularities on human skin, comprising the steps consisting in:
(a) applying topically to the skin a composition comprising, in a physiologically acceptable medium, at least 20% by weight of urea,
(b) leaving the composition in contact with the skin for a time of between 5 minutes and 30 minutes, and
(c) removing the composition by rinsing.

2. Process according to Claim 1, **characterized in that** the composition contains from 20% to 50% by weight of urea.

3. Process according to any one of Claims 1 to 2, **characterized in that** the composition contains a continuous aqueous phase.

4. Process according to Claim 3, **characterized in that** the composition also contains at least one compound chosen from alcohols and polyols containing from 1 to 3 carbon atoms.

5. Process according to Claim 4, **characterized in that** the said compound is chosen from ethanol, isopropanol, glycerol and propylene glycol.

6. Process according to any one of Claims 3 to 5, **characterized in that** the composition also contains at least one thickener.

7. Process according to Claim 6, **characterized in that** the said thickener is chosen from xanthan gum, an optionally crosslinked acrylic acid homopolymer or copolymer, a polyacrylamide, an acrylamidomethylpropanesulphonic acid homopolymer or copolymer, and cellulose derivatives.

8. Process according to any one of Claims 1 to 7, **characterized in that** it also comprises a preliminary step of applying to the skin a composition containing, in a physiologically acceptable medium, from 0.5% to 10% by weight of urea, before performing step (a).

9. Process according to any one of Claims 1 to 8, **characterized in that** it also comprises an additional step of applying to the skin a composition containing, in a physiologically acceptable medium, from 0.5% to 10% by weight of urea, after performing step (c).

10. Process according to any one of Claims 1 to 9, **characterized in that** it is intended for attenuating wrinkles and fine lines and/or pigmentation marks and/or acne scars and/or for unblocking skin pores.

11. Process according to any one of Claims 1 to 10, **characterized in that** the composition is applied to the face and/or the neck and/or the neckline and/or the hands and/or the back.

## Patentansprüche

1. Kosmetisches Verfahren für die Behandlung sichtbarer und/oder fühlbarer Unregelmäßigkeiten menschlicher Haut, das die folgenden Schritte umfasst:
(a) eine Zusammensetzung, die in einem physiologisch akzeptablen Medium mindestens 20 Gew.-% Harnstoff enthält, wird topisch auf die Haut aufgebracht,
(b) die Zusammensetzung wird für eine Zeitspanne von 5 bis 30 Minuten mit der Haut in Kontakt belassen, und
(c) die Zusammensetzung wird durch Spülen entfernt.

2. Verfahren nach Anspruch 1, **dadurch gekennzeichnet, dass** die Zusammensetzung 20 bis 50 Gew.-% Harnstoff enthält.

3. Verfahren nach einem der Ansprüche 1 bis 2, **dadurch gekennzeichnet, dass** die Zusammensetzung eine kontinuierliche wässrige Phase enthält.

4. Verfahren nach Anspruch 3, **dadurch gekennzeichnet, dass** die Zusammensetzung ferner mindestens eine Verbindung enthält, die unter den Alkoholen und Polyolen ausgewählt ist, die 1 bis 3 Kohlenstoffatome aufweisen.

5. Verfahren nach Anspruch 4, **dadurch gekennzeichnet, dass** der Alkohol unter Ethanol, Isopropanol, Glycerin und Propylenglycol ausgewählt ist.

6. Verfahren nach einem der Ansprüche 3 bis 5, **dadurch gekennzeichnet, dass** die Zusammensetzung außerdem ein Verdickungsmittel enthält.

7. Verfahren nach Anspruch 6, **dadurch gekennzeichnet, dass** das Verdickungsmittel ausgewählt ist unter Xanthangummi, einem gegebenenfalls vernetzten Acrylsäurehomopolymer oder Acrylsäurecopolymer, einem Polyacrylamid, einem Acrylamidomethylpropansulfonsäurehomopolymer oder Acrylamidomethylpropansulfonsäurecopolymer und Cellulosederivaten.

8. Verfahren nach einem der Ansprüche 1 bis 7, **dadurch gekennzeichnet, dass** es ferner einen vorbereitenden Schritt umfasst, in dem eine Zusammensetzung, die in einem physiologisch akzeptablen Medium 0,5 bis 10 Gew.-% Harnstoff enthält, auf die Haut aufgetragen wird, bevor Schritt (a) durchgeführt wird.

9. Verfahren nach einem der Ansprüche 1 bis 8, **dadurch gekennzeichnet, dass** es ferner einen zusätzlichen Schritt umfasst, in dem eine Zusammensetzung, die in einem physiologisch akzeptablen Medium 0,5 bis 10 Gew.-% Harnstoff enthält, auf die Haut aufgetragen wird, nachdem Schritt (c) durchgeführt wurde.

10. Verfahren nach einem der Ansprüche 1 bis 9, **dadurch gekennzeichnet, dass** es dafür vorgesehen ist, Falten und feine Linien zu mildern und/oder Pigmentflecken und/oder Aknenarben abzuschwächen und/oder Hautporen zu öffnen.

11. Verfahren nach einem der Ansprüche 1 bis 10, **dadurch gekennzeichnet, dass** die Zusammensetzung auf das Gesicht und/oder den Hals und/oder das Dekolleté und/oder die Hände und/oder den Rücken aufgebracht wird.

## Revendications

1. Procédé cosmétique de traitement des irrégularités visibles et/ou tactiles sur la peau humaine, comprenant les étapes consistant à :
(a) appliquer par voie topique sur la peau une composition comprenant, dans un milieu physiologiquement acceptable, au moins 20 % en poids d'urée,
(b) laisser la composition en contact avec la peau pendant une période comprise entre 5 minutes et 30 minutes, et
(c) éliminer la composition par rinçage.

2. Procédé selon la revendication 1, **caractérisé en ce que** la composition contient de 20 % à 50 % en poids d'urée.

3. Procédé selon l'une ou l'autre des revendications 1 et 2, **caractérisé en ce que** la composition contient une phase aqueuse continue.

4. Procédé selon la revendication 3, **caractérisé en ce que** la composition contient également au moins un composé choisi parmi les alcools et les polyols contenant de 1 à 3 atomes de carbone.

5. Procédé selon la revendication 4, **caractérisé en ce que** ledit composé est choisi parmi l'éthanol, l'isopropanol, le glycérol et le propylèneglycol.

6. Procédé selon l'une quelconque des revendications 3 à 5, **caractérisé en ce que** la composition contient également au moins un épaississant.

7. Procédé selon la revendication 6, **caractérisé en ce que** ledit épaississant est choisi parmi la gomme xanthane, un homopolymère ou copolymère d'acide acrylique éventuellement réticulé, un polyacrylamide, un homopolymère ou copolymère d'acide acrylamidométhylpropanesulfonique, et des dérivés de la cellulose.

8. Procédé selon l'une quelconque des revendications 1 à 7, **caractérisé en ce qu'**il comprend également une étape préliminaire d'application sur la peau d'une composition contenant, dans un milieu physiologiquement acceptable, de 0,5 % à 10 % en poids d'urée, avant d'effectuer l'étape (a).

9. Procédé selon l'une quelconque des revendications 1 à 8, **caractérisé en ce qu'**il comprend également une étape supplémentaire d'application sur la peau d'une composition contenant, dans un milieu physiologiquement acceptable, de 0,5 % à 10 % en poids d'urée, avant d'effectuer l'étape (c).

10. Procédé selon l'une quelconque des revendications 1 à 9, **caractérisé en ce qu'**il est destiné à atténuer les rides et les ridules et/ou les tâches pigmentaires et/ou les cicatrices acnéiques et/ou à déboucher les pores cutanés.

11. Procédé selon l'une quelconque des revendications 1 à 10, **caractérisé en ce que** la composition est appliquée sur le visage et/ou le cou et/ou le décolleté et/ou les mains et/ou le dos.
